Europäisches Patentamt

European Patent Office

Office européen des brevets

(19)

(11) Numéro de publication: **0 026 022**
**B1**

(12) **FASCICULE DE BREVET EUROPEEN**

(45) Date de publication du fascicule du brevet: **08.08.84**

(51) Int. Cl.³: **A 61 B 17/16**

(21) Numéro de dépôt: **80200881.3**

(22) Date de dépôt: **18.09.80**

(54) Perceuse-taraudeuse.

(30) Priorité: **24.09.79 FR 7924137**

(43) Date de publication de la demande:
**01.04.81 Bulletin 81/13**

(45) Mention de la délivrance du brevet:
**08.08.84 Bulletin 84/32**

(84) Etats contractants désignés:
**AT BE CH DE FR GB IT LI LU NL SE**

(56) Documents cités:
**FR - A - 2 374 886**
**GB - A - 319 607**
**GB - A - 751 618**
**GB - A - 934 284**
**US - A - 3 506 277**

(73) Titulaire: **Leuenberger, Roland**
**15, chemin du Bois de la Chapelle**
**CH-1219 Onex (CH)**

(72) Inventeur: **Leuenberger, Roland**
**15, chemin du Bois de la Chapelle**
**CH-1219 Onex (CH)**

(74) Mandataire: **Moinas, Michel**
**Moinas & Cie 42, rue Plantamour**
**CH-1201 Genève (CH)**

Courier Press, Leamington Spa, England.

## Description

La présente invention concerne une perceuse-taraudeuse à usages multiples, qui présente un intérêt tout particulier en chirurgie.

On connaît la nécessité, en chirurgie osseuse par exemple, de perceuses ayant des mouvements doux et de faible amplitude, de façon à ne pas risquer d'endommager les tissus voisins. En particulier, il convient d'éviter que, par dérapage sur l'os ou celui-ci ayant été traversé de part en part, l'outil de la perceuse ou de la taraudeuse vienne en contact avec les muscles. Dans un tel cas, les fibres musculaires s'enrouleraient aussitôt autour de l'outil et se déchireraient, ce qui conduirait à des dommages irréparables.

Une perceuse de ce type, à mouvement de rotation alternatif, est décrite dans le brevet suisse n° 610 753.

Percer et tarauder ou tarauder et percer sont des opérations qui vont souvent de pair. On comprend dès lors l'intérêt qu'il y aurait à pouvoir utiliser un seul et même instrument pour les deux opérations, tout en gardant les critères de mouvements doux et de faible amplitude.

L'invention résoud ce problème technique et présente les avantages énoncés ci-dessus. Par simple déplacement d'un organe de commande en effet, l'instrument selon l'invention peut faire office de perceuse ou de taraudeuse. Cette perceuse-taraudeuse présente d'ailleurs un intérêt général, de par le mouvement communiqué à l'outil, et contribue par exemple à la sécurité dans le bâtiment ou en usine et à la prévention des accidents de bricolage.

La perceuse-taraudeuse selon l'invention comporte un arbre moteur destiné à être accouplé à des moyens d'oscillation autour de son axe, un porte-outil et un mécanisme d'accouplement en rotation reliant l'arbre moteur au porte-outil caractérisée en ce que ledit mécanisme d'accouplement en rotation comprend:

— une pièce d'entraînement intérieure munie de butées périphériques se succédant axialement et dont les flancs d'appui sont orientés en sens opposés, parallélement à l'axe de l'arbre moteur ladite pièce comportant trois parties

— une première partie où les flancs opposés des butées sont disposés en vis-à-vis,

— une deuxième et une troisième parties où de manière alternée un dégagement est ménagé en vis-à-vis d'un flanc d'une butée,

— une pièce d'entraînement extérieure tubulaire pouvant coulisser axialement sur la pièce intérieure,

— un organe d'accouplement de la pièce intérieure avec la pièce extérieure solidaire de ladite pièce extérieure, coopérant avec au moins une des butées de la pièce intérieure, et pouvant glisser librement dans les dégagements.

Dans un mode de réalisation, la perceuse comporte un organe de commande servant à déplacer axialement l'organe d'accouplement devant les flancs opposés des butées de manière soit à transmettre directement les oscillations de l'arbre moteur, soit à transformer celles-ci en un mouvement de rotation unidirectionnel par impulsions successives dans un sens ou dans l'autre.

Dans une forme d'exécution, l'organe d'accouplement est un doigt à ressort traversant la pièce annulaire extérieure, solidaire du porte-outil, ce doigt prenant appui dans une rainure axiale ménagée dans la pièce intérieure, solidaire de l'arbre moteur.

Avantageusement, l'organe de commande prend appui avec jeu dans une rainure annulaire ménagée dans la pièce extérieure.

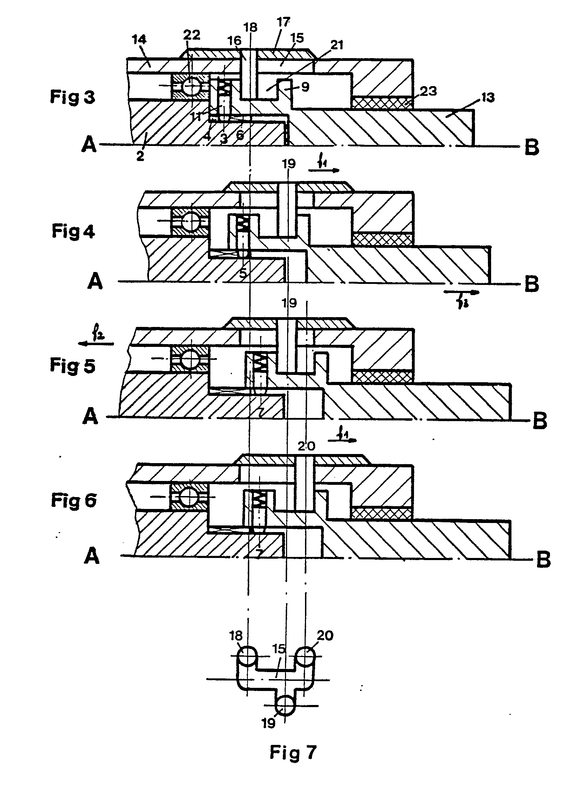

L'invention sera mieux comprise en référence au dessin annexé donné à titre d'exemple. Pour la clarté des figures, ne sont reportées d'une figure à l'autre, à partir de la figure 3, que les références correspondant à des éléments nouveaux d'une figure à l'autre ou nécessaire aux explications.

La fig. 1 est une vue schématique éclatée en perspective d'une forme d'exécution simple.

La fig. 2 est un schéma de principe des mouvements. Les fig. 3 à 6 sont des demi-coupes d'une forme d'exécution plus élaborée, dans les différentes configurations possibles. Par rapport à la fig. 1, les fig. 3 à 6 correspondraient à une vue de l'arrière en coupe.

La fig. 7 est une vue de dessus d'une rainure avec positions de verrouillage, ménagée dans le carter représenté aux fig. 3 à 6..

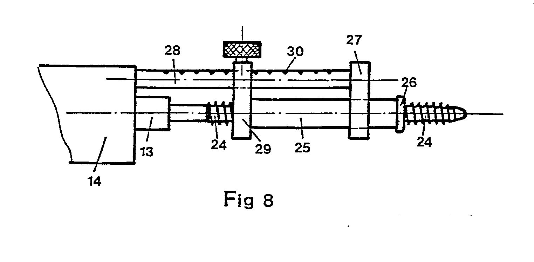

Enfin la fig. 8 est une vue de côté d'une forme d'exécution avec butée.

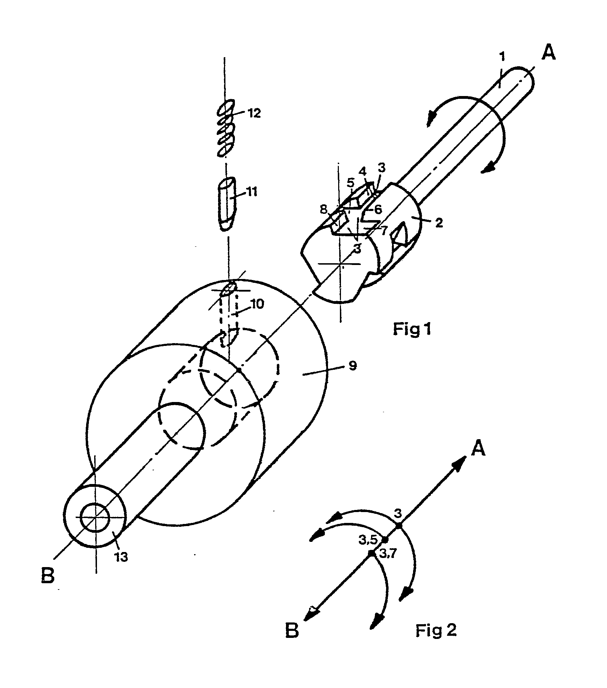

Dans la forme d'exécution représentée à la fig. 1, un organe moteur non-représenté se trouve en haut à droite, côté A, et communique à l'arbre 1 un mouvement de rotation alternatif ou oscillant, comme symbolisé par la flèche aller-retour. Une pièce intérieure ou centrale 2 est solidaire de l'arbre moteur 1.

Dans cette pièce 2, on distingue successivement trois parties:

— une première partie qui présente une rainure 3 et uniquement une rainure 3; les flancs de cette rainure 3 sont symbolisés par 4;

— une seconde partie dans laquelle la rainure 3 se prolonge, mais est flanquée d'un dégagement 5 sur un côté; l'unique flanc de la rainure est symbolisé par 6;

— une troisième partie dans laquelle la rainure 3 se prolonge, mais est flanquée d'un dégagement 7 sur l'autre côté; l'unique flanc de la rainure est symbolisé par 8.

Les deuxième et troisième partie de la pièce centrale 2 présentent les caractéristiques d'une came et correspondant à deux cames de sens contraire juxtaposées.

A cette pièce centrale 2 est assujettie une pièce extérieure 9, solidaire d'un porte-outil 13, côté B; cette pièce 9, tubulaire présente un perçage radial 10 qui reçoit un doigt 11 muni d'un ressort 12. Ce doigt 11, prenant appui dans la rainure 13 de la pièce centrale 2 fait office de cliquet. Ce doigt 11 peut se déplacer le long de la rainure 3 et glisser librement dans les dégagements 5 et 7. De préférence, l'extrémité de ce doigt 11 présente une conicité et la rainure 3 une ouverture correspondante. Sur cette fig. 1, le carter extérieur n'est pas représenté.

On conçoit aisément que, selon la position du doigt 11 dans la rainure 3, le mouvement communiqué au porte-outil 13 va être le même mouvement que celui de l'arbre moteur 1, soit un mouvement de rotation alternatif, si le doigt est en position 3 en prenant appui sur les deux flancs 4 de la rainure 3, tandis que le mouvement de rotation alternatif de l'arbre moteur sera transformé sur le porte-outil en un mouvement de rotation par impulsions successives ou à-coups, dans un seul sens, si le doigt 11 est positionné dans la rainure 3 en regard du dégagement 5 et ne prend ainsi appui que sur le flanc 6. Si le doigt 11 a avancé encore d'un cran et est positionné dans la rainure 3 en regard du dégagement 7, le mouvement communiqué est un mouvement de rotation par impulsions successives ou à-coups, dans le sens contraire.

Tout ceci est représenté schématiquement dans la fig. 2, où les chiffres 3, respectivement 3,5 et 3,7 représentent les positions du doigt 11 et les flèches traduisent le mouvement communiqué.

Dans l'autre forme d'exécution, plus élaborée, représentée aux fig. 3 à 6, l'arbre moteur 1 non-représenté est placé à gauche, du côté A, tandis que le porte-outil 13, représenté sans le logement pour l'outil, est placé à droite, du côté B.

Sur ces demi-coupes, la pièce tubulaire 9 présente une rainure annulaire 21, le tout étant entouré par un carter 14, immobile, en rotation qui est en contact avec les éléments en mouvement par l'intermédiaire de roulements ou paliers 22 et de coussinets 23.

Ce carter 14 présente une rainure axiale 15 avec trois positions de verrouillage 18, 19 et 20, comme représenté vu de dessus à la fig. 7. Cette rainure 15 est traversée par un organe de commande 16 fixé sur une bague extérieure 17 et qui coopère avec la rainure annulaire 21 ménagée dans la pièce tubulaire 9. Cet organe de commande 16 est susceptible de se verrouiller dans la rainure 15 en position 18, 19 ou 20.

En variante avantageuse, permettant comme décrit plus bas l'inversion du mouvement de rotation par impulsions successives simplement en poussant ou en tirant sur la perceuse-taraudeuse, la distance entre la première position de verrouillage 18, côté arbre moteur, et la seconde position de verrouillage 19 est égale à la course du doigt 11 d'accouplement dans la rainure 3 de la pièce centrale 2; la distance entre la deuxième position de verrouillage 19 et la troisième position de verrouillage 20 est la moitié de la distance entre la première position de verrouillage 18 et la deuxième position de verrouillage 19. Ces proportions sont respectées sur la fig. 7.

En référence aux figures, la perceuse-taraudeuse remplit successivement les différentes fonctions de percer, par un mouvement de rotation oscillant, de visser ou tarauder par un mouvement de rotation par impulsions successives dans le sens du vissage, de dévisser ou de sortie du taraud par un mouvement semblable de sens contraire, le passage de la fonction "vissage" ou "taraudage" à la fonction "dévissage" ou "sortie du taraud" pouvant se réaliser par simple poussée sur la perceuse-taraudeuse, ou retrait de celle-ci.

Dans la fig. 3, le doigt 11 d'accouplement est dans la rainure 3 et vient buter de chaque côté sur les flancs 4. Le mouvement transmis est alors celui de l'arbre moteur, c.à.d. un mouvement de rotation oscillant. L'organe de commande 16 est en position de verrouillage 18.

En déplaçant la bague 17 dans la direction de B comme représenté par la flèche $f_1$, l'organe de commande 16 vient buter contre la face interne avant de la rainure 21, puis poursuivant sa course pour se verrouiller en position 19, déplace la pièce tubulaire 9 et le porte-outil 13, ainsi que le doigt 11 d'accouplement qui vient se placer en position 5 de la rainure 3. Ce doigt 11 ne peut plus buter que sur la face 6 de la rainure 3 et on comprend aisément que le mouvement d'oscillation est "redressé" en un mouvement de rotation dans un sens, mais par impulsions successives. Cette configuration (fig. 4) correspond par exemple à la fonction "taraudage" ou "vissage".

Si maintenant, sans toucher à la bague 17, on exerce une force de retrait vers A, tel que représenté par la flèche $f_2$, on aboutit à la configuration représenté à la fig. 5. L'organe de commande 16 n'a évidemment pas bougé par rapport au carter 14 et est resté dans sa position 19. L'outil, soumis à des forces de vissage et au frottement, n'a pas bougé, et c'est donc l'ensemble arbre moteur 1, pièce centrale 2 et carter 14 qui ont reculé. Le doigt 11 d'accouplement, sans avoir bougé lui-même, se retrouve dans la position 7 de la rainure 3 et ne peut plus prendre appui que sur le flanc 8 de cette rainure 3, ce qui correspond à un mouvement de rotation par impulsions successives inverse du cas précédent. Cette configuration correspond par exemple à la fonction "sortie du taraud" ou "dévissage".

Une simple poussée sur l'arbre 1 et la pièce 2 dans la direction de l'outil la (flèche $f_3$) restitue la configuration représentée à la fig. 4 et inverse à nouveau le mouvement de rotation par impulsions successives. La fonction "taraudage" ou "vissage" est retrouvée.

Ainsi, par simple poussée en retrait de l'outil vis-à-vis de la pièce centrale 2 les mouvements de rotation peuvent être inversés.

Notons que sur les fig. 4 et 5, au contraire de ce qui se produit réellement, les éléments ne bougeant pas sous l'action des forces $f_2$ et $f_3$ ont été représentés déplacés, tandis que les éléments bougeant sous l'action des mêmes forces $f_2$ et $f_3$ ont été représentés non-déplacés. Cette représentation, à l'inverse de la réalité, permet de mieux comprendre les rapports de distance entre les positions successives du doigt 11 d'accouplement, soit 3, respectivement 3,5 et 3,7, et les positions de verrouillage 18,19 et 20 de l'organe de commande 16.

Dans la fig. 6, la bague a été déplacée dans la direction de la flèche $f_1$ et l'organe de commande 16 s'est placé en position de verrouillage 20. Cette configuration donne le même résultat que celle qui est représentée à la fig. 5, par exemple la fonction "sortie de taraud" ou "dévissage", à ceci près que le mécanisme est verrouillé et que le mouvement ne peut plus être inversé par simple retrait de l'outil vis-à-vis de la pièce centrale.

Dans un mode de réalisation avantageux, l'arbre moteur transmet un mouvement de rotation oscillant d'amplitude inférieure à un demi tour, par exemple légèrement supérieure à 120°. Le mécanisme d'accouplement peut alors comprendre un doigt et une pièce centrale qui présente trois rainures au tour, disposées régulièrement. En variante préférée, le mécanisme d'accouplement comprend deux doigts et la pièce centrale présente six rainures au tour, disposées régulièrement.

Une telle perceuse-taraudeuse peut former un tout et être vendue pour elle-même.

En variante, elle peut comprendre deux parties, qui peuvent s'acquérir séparément:
— une première partie, jouant le rôle d'arbre moteur, constituée d'une perceuse à mouvement de rotation oscillant comme celle décrite dans le brevet suisse no 610 753 cité en début d'exposé.
— une seconde partie, adaptable à la première et servant d'accessoire, comprenant le mécanisme d'accouplement et le porte-outil.

Une variante particulièrement avantageuse, notamment pour tarauder (et visser), correspond à la fig. 8, où on a représenté, en vue de profil et sur la gauche, le porte-outil 13 et le carter 14 (côté B des fig. 3 à 6). Sur le porte-outil 13 est monté un taraud 24 qui traverse une tige creuse 25 terminée par une butée 26. Cette tige 25 est supportée par une pièce 27 qu'elle traverse et par une tige 28 fixée au carter 14. Sur cette même tige 25 est fixé un organe 29 qui coulisse sur la tige 28 et sert au réglage de la position de la butée 26, ce réglage étant assuré de préférence par une bille non visible, serrée dans une des empreintes 30 de la tige 28. La butée 26 est ainsi positionnée, en référence à une échelle graduée, de façon à laisser dépasser le taraud 24 de la longueur à tarauder.

Partant de la position taraudage de la fig. 4, on voit que, quand la butée 26 est retenue, le taraud, en continuant d'avancer dans le filet qu'il a taillé, tire le porte-outil 13 vers l'avant et l'amène dans la position de la fig. 5. Le sens de rotation est inversé, le doigt 11 d'accouplement passant de la position 3,5 à 3,7. Ce faisant, le taraud repousse maintenant le porte-outil 13 qui revient dans la position de la fig. 4. Le sens de rotation est à nouveau inversé, le doigt 11 d'accouplement revenant en position 3,5, ... et ainsi de suite.

Résultat, le taraud 24, en butée, va se mettre à osciller sans blesser le filet et sans dépasser la longueur fixée d'avance par l'organe 29 de réglage.

La sécurité est donc totale.

**Revendications**

1. Perceuse-taraudeuse comportant un arbre moteur (1), destiné à être accouplé à des moyens d'oscillation autour de son axe, un porte-outil (13) et un mécanisme d'accouplement en rotation (2, 9, 11) reliant l'arbre moteur (1) au porte-outil (13) caractérisée en ce que ledit mécanisme d'accouplement en rotation comprend:
— une pièce d'entraînement intérieure (2) munie de butées périphériques se succédant axialement et dont les flancs d'appui (4, 6, 8) sont orientés en sens opposés, parallélement à l'axe de l'arbre moteur ladite pièce comportant trois parties:
— une première partie où les flancs opposés (4) des butées sont disposés en vis-à-vis,
— une deuxième et une troisième parties où de manière alternée un dégagement (5, 7) est ménagé en vis-à-vis d'un flanc (6, 8) d'une butée,
— une pièce d'entraînement extérieure tubulaire (9) pouvant coulisser axialement sur la pièce intérieure (2),
— un organe d'accouplement (11) de la pièce intérieure (2) avec la pièce extérieure (9) solidaire de ladite pièce extérieure, coopérant avec au moins une des butées (4, 6, 8) de la pièce intérieure (2), et pouvant glisser librement dans les dégagements (5, 7).

2. Perceuse-taraudeuse selon la revendication 1 caractérisé en ce qu'elle comporte un organe de commande (16) servant à déplacer axialement l'organe d'accouplement (11) devant les flancs opposés des butées de manière soit à transmettre directement les oscillations de l'arbre moteur, soit à transformer celles-ci en un mouvement de rotation unidirectionnel par impulsions successives dans un sens et/ou dans l'autre.

3. Perceuse-taraudeuse selon l'une des revendications 1 et 2 caractérisée en ce que l'organe d'accouplement est un doigt (11) à ressort (12) traversant la pièce extérieure tubulaire (9), solidaire du porte-outil (13) ce doigt (11) prenant appui dans une rainure (3) axiale ménagée dans la pièce intérieure (2), solidaire de l'arbre moteur (1).

4. Perceuse-taraudeuse selon la revendication 3, caractérisée par le fait que le doigt (11) à ressort (12) présente à son extrémité une conicité et que les parois (4, 6 et 8) de la rainure (3) présentent un même angle d'ouverture.

5. Perceuse-taraudeuse selon la revendication 3, caractérisée par le fait que l'amplitude du mouvement d'oscillation communiqué par l'arbre moteur est comprise entre 120° et 180° et que le mécanisme d'accouplement comprend un seul doigt (11), la pièce intérieure (2) présentant trois rainures (3) au tour, disposées régulièrement.

6. Perceuse-taraudeuse selon la revendication 3, caractérisée par le fait que l'amplitude du mouvement d'oscillation est comprise entre 120° et 180° et que le mécanisme d'accouplement comprend deux doigts (11), la pièce intérieure (2) présentant six rainures (3) au tour, disposées régulièrement.

7. Perceuse-taraudeuse selon la revendication 2, caractérisée par le fait que l'organe de commande (16) prend appui avec jeu dans une rainure annulaire (21) ménagée dans la pièce extérieure (9), en sorte que, pour la position de l'organe de commande (16) transformant les oscillations en impulsions dans un seul sens et sans toucher à celui-ci, le sens de rotation est inversé en modifiant les positions respectives des pièces intérieure (2) et extérieure (9).

8. Perceuse-taraudeuse selon la revendication 7, caractérisée par le fait que l'organe de commande (16) est fixé sur un bague (17) qui glisse sur un carter (14) entourant le mécanisme d'accouplement et qui traverse ce carter (14) par un perçage axial (15) ménagé dans celui-ci et comportant trois positions de verrouillage (18, 19, 20).

9. Perceuse-taraudeuse selon la revendication 8, caractérisée par le fait que la distance entre la première position de verrouillage (18), côté arbre moteur, et la seconde position de verrouillage (19) est égale à la course du doigt (11) d'accouplement dans la rainure (3) de la pièce intérieure (2).

10. Perceuse-taraudeuse selon la revendication 9, caractérisée par le fait que la distance entre la seconde position de verrouillage (19) et la troisième position de verrouillage (20) est la moitié de la distance entre la première position de verrouillage (18) et la seconde position de verrouillage (19).

11. Perceuse-taraudeuse selon la revendication 8, caractérisée par le fait que l'arbre moteur (1), la pièce intérieure (2), la bague (17) portant l'organe de commande (16), la pièce extérieure (9) et le porte-outil (13) sont coaxiaux, le doigt (11) d'accouplement étant perpendiculaire à l'axe.

12. Perceuse-taraudeuse selon la revendication 8, caractérisée par le fait qu'elle comporte une butée (26) solidaire du carter (14).

13. Perceuse-taraudeuse selon la revendication 12, caractérisée par le fait que la butée (26) est réglable en distance par rapport au carter (14).

**Claims**

1. A combined drilling-tapping device comprising a drive shaft (1) designed to be coupled to means for providing an oscillatory movement about its axis, a tool carrier (13) and a mechanism for rotational coupling (2,9,11) of the drive shaft (1) to the tool carrier (13), characterised in that the said rotational coupling mechanism comprises: an inner drive member (2) provided with peripheral stops which follow on from one another axially and of which the bearing faces (4,6,8) are orientated in opposite directions parallel with the axis of the drive shaft, the said member comprising three parts:
— a first part in which the opposite faces (4) of the abutments are disposed face-to-face,
— a second and a third part where a clearance (5,7) is alternately provided facing one face (6,8) of a stop,
— a tubular outer drive member (9) capable of sliding axially on the inner member (2),
— a means (11) for coupling the inner member (2) to the outer member (9) rigid with the said outer member and co-operating with at least one of the stops (4,6,8) of the inner member (2) and capable of sliding freely in the clearances (5,7).

2. A combined drilling-tapping device according to Claim 1, characterised in that it comprises an operating means (16) for axial displacement of the coupling member (11) in front of the oppositely disposed faces of the stops in such a manner as directly to transmit the oscillating movements of the drive shaft, in other words so that it can convert these movements into a unidirectional rotary movement by successive pulses in one direction and/or in the other.

3. Combined drilling-tapping device according to either of Claims 1 or 2, characterised in that the coupling member is a dog (11) having a spring (12) which traverses the tubular outer member (9) and which is rigid with the tool holder (13), the said dog (11) being supported in an axial groove (3) provided in the inner member (2), rigid with the drive shaft (1).

4. Combined drilling-tapping device according to Claim 3, characterised in that the dog (11) which has the spring (12) has at its end a conical shape and in that the walls (4, 6 and 8) of the groove (3) have one and the same angle of opening.

5. Combined drilling-tapping device according to Claim 3, characterised in that the magnitude of the oscillatory movement communicated by the drive shaft is comprised between 120° and 180° and in that the coupling mechanism comprises a single dog (11), the inner member (2) having three grooves (3) disposed regularly around it.

6. Combined drilling-tapping device according to Claim 3, characterised in that the magnitude of the oscillatory movement is comprised between 120° and 180° and in that the coupling mechanism comprises two dogs (11) the inner member (2) having six grooves (3) disposed regularly around it.

7. Combined drilling-tapping device according to Claim 2, characterised in that the operating means (16) bears with clearance in an annular groove (21) disposed in the outer member (9), so that for the position of the operating member (16) which converts the oscillatory movements into pulses in just one direction and without touching this latter, the direction of rotation is reversed by modifying the respective positions of the inner member (2) and outer member (9).

8. Combined drilling-tapping device according to Claim 7, characterised in that the operating member (16) is fixed on a ring (17) which slides on a casing (14) surrounding the coupling mechanism and which traverses the said casing (14) via an axial bore (15) provided in the latter and comprising three locking positions (18,19,20).

9. Combined drilling-tapping device according to Claim 8, characterised in that the distance between the first locking position (18), on the drive shaft side, and the second locking position (19) is equal to the travel of the coupling dog (11) in the groove (3) of the inner member (2).

10. Combined drilling-tapping device according to Claim 9, characterised in that the distance between the second locking position (19) and the third locking position (20) is half the distance from the first locking position (18) to the second locking position (19).

11. Combined drilling-tapping device according to Claim 8, characterised in that the drive shaft (1), the inner member (2), the ring (17) carrying the oscillating member (16), the outer member (9) and the tool carrier (13) are coaxial, the coupling dog (11) being perpendicular to the axis.

12. Combined drilling-tapping device according to Claim 8, characterised in that it comprises a stop (26) rigid with the casing (14).

13. Combined drilling-tapping device according to Claim 12, characterised in that the stop (26) is regulable in its distance from the casing (14).

**Patentansprüche**

1. Bohr- und Gewindeschneidmaschine mit einer Antriebswelle (1), die mit Mitteln zur Schwingungserzeugung um seine Achse koppelbar ist, mit einem Werkzeugfutter (13), und mit einer Vorrichtung zur drehschlüssigen Verbindung (2, 9, 11) der Antriebswelle (1) mit dem Werkzeugfutter (13), dadurch gekennzeichnet, dass die Vorrichtung zur drehschlüssigen Verbindung

— einen Innenmitnehmer (2), der am Umfang miteinander axial aufeinanderfolgenden Anschlägen versehen ist, deren Angriffsflanken (4, 6, 8) im Gegensinn parallel zur Antriebswellenachse angebracht sind, und wobei der Mitnehmer drei Bereiche aufweist:

— einen ersten Bereich, wo sich die im Gegensinn angebrachten Angriffsflanken (4) gegenüberstehen,

— zweite und dritte Bereiche, wo Ausnehmungen (5, 7) mit einer der gegenüberliegenden Angriffsflanken (6, 8) eines Anschlags abwechseln,

— einen axial auf dem Innenmitnehmer gleitfähigen rohrförmigen Aussenmitnehmer (9),

— ein Kupplungsorgan (11) des Innenmitnehmers (2) mit dem Aussenmitnehmer (9), das mit dem Aussenmitnehmer verbunden ist und mit mindestens einem der Anschläge (4, 6, 8) des Innenmitnehmers (2) zusammenwirkt und frei in den Ausnehmungen (5, 7) gleiten kann, aufweist.

2. Bohr- und Gewindeschneidmaschine nach Anspruch 1, dadurch gekennzeichnet, dass sie ein Bedienungsorgan (16) aufweist, welches zur axialen Verschiebung des Kupplungsorgans (11) vor den gegenüberliegenden Anschlägen dient, um entweder die Schwingungen der Antriebswelle direkt zu übertragen oder diese durch aufeinanderfolgende, einseitige und/oder zweiseitig gerichtete Impulse in eine einsinnige Drehbewegung umzusetzen.

3. Bohr- und Gewindeschneidmaschine nach Anspruch 1 oder 2, dadurch gekennzeichnet, dass das Kupplungsorgan ein durch eine Feder (12) belasteter Finger (11) ist, der den rohrförmigen, mit dem Werkzeugfutter verbundenen Aussenmitnehmer (9) durchsetzt, wobei sich der Finger (11) in einer Axialnut (3) im Innenmitnehmer (2) abstützt, der mit der Antriebswelle verbunden ist.

4. Bohr- und Gewindeschneidmaschine nach Anspruch 3, dadurch gekennzeichnet, dass der mit der Feder (12) belastete Finger (11) am Ende konisch verjüngt ist, und dass die Flanken (4, 6 und 8) der Nut (3) einen gleichen Oeffnungswinkel bilden.

5. Bohr- und Gewindeschneidmaschine nach Anspruch 3, dadurch gekennzeichnet, dass die Amplitude der von der Antriebswelle mitgeteilten Schwingbewegung zwischen 120° und 180° liegt, und dass die Kupplungsvorrichtung einen einzigen Finger (11) aufweist, wobei der Innenmitnehmer (2) am Umfang drei regelmässig angeordnete Nuten (3) besitzt.

6. Bohr- und Gewindeschneidmaschine nach Anspruch 3, dadurch gekennzeichnet, dass die Amplitude der Schwingbewegung 120° bis 180° beträgt, und dass die Kupplungsvorrichtung zwei Finger (11) aufweist, wobei der Innenmitnehmer am Umfang sechs regelmässig angeordnete Nuten besitzt.

7. Bohr- und Gewindeschneidmaschine nach Anspruch 2, dadurch gekennzeichnet, dass sich das Bedienungsorgan (16) mit Spiel in einer im Aussenmitnehmer (9) angebrachten Ringnut (21) derart abstützt, dass in der Stellung des Bedienungsorgans (16), wo die Schwingungen in einsinnige Impulse umgesetzt werden, ohne Berührung des Organs der Drehsinn dadurch umgekehrt wird, dass die gegenseitige Lage von Innenmitnehmer (2) zu Aussenmitnehmer (9) geändert wird.

8. Bohr- und Gewindeschneidmaschine nach Anspruch 7, dadurch gekennzeichnet, dass das Bedienungsorgan (16) auf einem Ring (17) angebracht ist, der auf einer das Kupplungsorgan umgebenden Hülse (14) gleitet, sie in einer axialen Bohrung (15) der Hülse durchsetzt und drei Verriegelungsstellungen (18, 19, 20) aufweist.

9. Bohr- und Gewindeschneidmaschine nach Anspruch 8, dadurch gekennzeichnet, dass der Abstand zwischen der ersten, antriebswellenseitigen Verriegelungsstellung (18) und der zweiten Verriegelungsstellung (19) gleich dem Verschiebungsweg des Kupplungsfingers (11) in der Nut (3) des Innenmitnehmers (2) ist.

10. Bohr- und Gewindeschneidmaschine nach Anspruch 9, dadurch gekennzeichnet, dass der Abstand zwischen der zweiten (19) und der dritten Verriegelungsstellung (20) halb so gross wie der Abstand zwischen erster (18) und zweiter Verriegelungsstellung ist.

11. Bohr- und Gewindeschneidmaschine nach Anspruch 8, dadurch gekennzeichnet, dass die Antriebswelle (1), der Innenmitnehmer (2), der das Bedienungsorgan (16) tragende Ring (17), der Aussenmitnehmer (9) und das Werkzeugfutter (13) koaxial sind und der Kupplungsfinger (11) senkrecht zur Achse steht.

12. Bohr- und Gewindeschneidmaschine nach Anspruch 8, dadurch gekennzeichnet, dass an der Hülse (14) ein Anschlag (26) vorgesehen ist.

13. Bohr- und Gewindeschneidmaschine nach Anspruch 12, dadurch gekennzeichnet, dass der Anschlag (26) in seinem Abstand von der Hülse (14) einstellbar ist.

Fig 1

Fig 2

Fig 3

Fig 4

Fig 5

Fig 6

Fig 7

Fig 8